(19) Europäisches Patentamt
European Patent Office
Office européen des brevets

(11) **EP 3 560 550 A1**

(12) **EUROPEAN PATENT APPLICATION**

(43) Date of publication:
**30.10.2019 Bulletin 2019/44**

(21) Application number: **18172976.5**

(22) Date of filing: **17.05.2018**

(51) Int Cl.:
*A61N 1/08* (2006.01)          *A61N 1/05* (2006.01)
*G01R 33/28* (2006.01)        *H01B 7/04* (2006.01)
*H03H 1/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2018  US 201862663286 P**

(71) Applicant: **BIOTRONIK SE & Co. KG**
**12359 Berlin (DE)**

(72) Inventors:
• **von Arx, Jeffrey A.**
  **Lake Oswego, OR 97035 (US)**

• **Shurig, Robert R.**
  **Tualatin, OR 97062 (US)**
• **Kurpad, Krishna K.N.**
  **Portland, OR 97229 (US)**
• **Sutton, Brian P.**
  **West Linn, OR 97068 (US)**
• **Brown, James E.**
  **Tigard, OR 97224 (US)**
• **Stadnik, Paul**
  **Lake Oswego, OR 97035 (US)**

(74) Representative: **Keck, Hans-Georg**
**Biotronik Corporate Services SE**
**Corporate Intellectual Property**
**Sieversufer 7 - 9**
**12359 Berlin (DE)**

(54) **NEUROMODULATION LEAD FOR REDUCING INTERACTIONS WITH MRI**

(57)    The present invention relates to a lead (1) comprising at least a first and a second conductor (12, 13), wherein the first conductor (11) and the second conductor (13) each comprise an electrically conducting core (100) that is surrounded by an electrical insulator (22, 23), wherein the electrical insulator (22) of the first conductor (12) consists of a first material (M1), and wherein the electrical insulator (23) of the second conductor (13) consists of a second material (M2), wherein the first material (M1) differs from the second material (M2). The first material (M1) and the second material (M2) are chosen such that the respective conductor (12, 13) comprises a resonance frequency that is different from a frequency used in an MRI device.

FIG. 1

**Description**

Conventional designs of percutaneous SCS leads are in general not suitable for MR-Conditional Full Body Scan (FBS) labeling.

MRI (Magnetic Resonance Imaging) devices put out very large RF (Radio Frequency) fields during operation. The energy in these fields is picked up by conductors in the SCS lead. If the leads do not have mechanisms for dissipating this energy, then the energy reaches the electrodes of the lead where it is dissipated as heat. Disadvantageously, such an electrode heating can damage tissue of the patient.

US 9,399,129 describes a medical lead having multiple conductors which are wound in an interleaved manner. Furthermore, US 2016/0331960 A1 describes different materials suited as electrical insulation for leads.

Based on the above, there is a desire for providing comparatively simple leads, particularly SCS lead, which do not suffer from electrode heating when subjected to MRI, and which are particularly MR conditional Full Body Scan.

This objective is solved by a lead having the features of claim 1. Particular embodiments of this aspect of the present invention are stated in the corresponding sub claims.

According to claim 1, a lead for transporting an electrical current is disclosed, comprising at least a first and a second conductor, wherein the first conductor and the second conductor each comprise an electrically conducting core (comprising e.g. at least one wire or several wires) that is surrounded by an electrical insulator, wherein the electrical insulator of the first conductor consists of a first material, and wherein the electrical insulator of the second conductor consists of a second material, wherein the first material differs from the second material.

The respective core can e.g. be formed by a single wire or a plurality of adjacent wires.

Advantageously, by using different materials for said electrical insulations, the present invention allows to tune (e.g. during the design phase) of each conductor's resonant frequency to e.g. ensure that none of the conductors are in resonance at the used or any useable MR frequencies depending on Lamor frequencies. Particular, in this way, the present invention allows to tune the individual conductor such that MR-Conditional FBS labeling is enabled.

According to an embodiment of the present invention, the first material and the second material are chosen such that the lead comprises a resonance frequency that is different from a frequency used in an MRI device, particularly different from 64 MHz (e.g. for 1.5 T MRI devices) or different from 128 MHz (e.g. for 3T MRI devices).

Furthermore, according to an embodiment of the present invention, the first material and the second material are selected from the group of the following materials: a ceramics, a polymer, ETFE, PFA, PTFE, polyimide, aluminum oxide, barium titanate, titanium dioxide.

Furthermore, according to an embodiment of the present invention, said conductors of the lead, e.g. the first and the second conductor, each form a helical coil.

According to a further embodiment, the respective conductor can also extend linearly. Furthermore, alternatively, two or more conductors of the lead can be braided or wound in an interleaved manner.

Further, according to an embodiment of the present invention, the lead comprises a plurality of conductors including said first and said second conductor, wherein each conductor of said plurality of conductors other than the first and the second conductor also comprises an electrically conducting core (e.g. at least one wire or several wires) that is surrounded by an electrical insulator formed out of the first material or out of the second material, or out of a further material different from the first and the second material.

Furthermore, according to an embodiment of the present invention, said plurality of conductors may consist of at least eight, or at least 16 conductors.

Furthermore, according to an embodiment of the present invention, the lead comprises a lead body insulator surrounding each individual conductor. The lead body insulator can be formed out of or can comprise polyurethane or silicone.

Furthermore, according to an embodiment of the present invention, the lead comprises at least one further conductor formed by a non-insulated (i.e. bare) electrically conducting member, which is electrically insulated with respect to its surrounding by means of electrical insulators of adjacent conductors of the lead and/or by means of said lead body insulator. Particularly that electrically conducting member may be formed by at least one or several wires.

Furthermore, according to an embodiment of the present invention, said first and said second conductor are co-radial and/or co-axial helical coils. Besides said first and second conductor also each remaining conductor (see above) can be a co-radial and/or a co-axial helical coil with respect to another conductor of the lead.

Particularly, two adjacent conductors form co-radial helical coils if they are arranged one inside the other and have the same radius and pitch. Further, particularly, two co-axial helical coils (or conductors) are co-axial in case they have a common coil axis around which the respective coil (or conductor) is wound.

Furthermore, according to an embodiment of the present invention, said conductors (e.g. the first and the second conductor and particularly the remaining conductors if present) form an inner coil structure and a co-axial outer coil structure surrounding the inner coil structure, wherein each coil structure comprises a plurality of conductors, each conductor forming a helical coil, wherein particularly the helical coils (conductors) of the inner coil structure are co-radial and/or wherein the helical coils (conductors) of the outer coil structure are co-radial.

Also here, particularly, two coil structures are co-axial in case they have a common coil axis around which the conductors of the respective coil structure are wound.

Furthermore, according to an embodiment of the present invention, the lead is a medical lead (i.e. a lead of a medical device). Particularly, the lead is an implantable medical lead.

Furthermore, according to an embodiment of the present invention, the lead is an electrode lead comprising a plurality of electrodes. Each electrode may form a section, particularly a circumferential section, of an outer surface of the lead, wherein each electrode is electrically connected to one of the conductors. Particularly the lead comprises at least two electrodes, particularly at least eight electrodes, particularly at least 16 electrodes.

Furthermore, according to an embodiment of the present invention, the lead is an SCS lead, i.e. a lead adapted for spinal cord stimulation (SCS).

According to a further aspect of the present invention, a method for producing a lead, particularly a lead according to the present invention, is disclosed, wherein at least a first and a second conductor are provided, wherein the first conductor and the second conductor each comprise an electrically conducting core (see e.g. above) that is surrounded by an electrical insulator, wherein the electrical insulator of the first conductor is made out of a first material, and wherein the electrical insulator of the second conductor is made out of a second material, wherein the first material differs from the second material, and wherein said conductors are surrounded by an outer lead body insulator.

The materials that can be used are already stated above.

Particularly, according to an embodiment of the method according to the present invention, the first material and the second material are chosen such that the respective conductor comprises a resonance frequency that is different from a pre-defined frequency used in an MRI device, particularly different from 64 MHz (e.g. for 1.5 T MRI devices) or different from 128 MHz (e.g. for 3T MRI devices), see also above.

Particularly, the invention allows providing a MR Conditional full body scan SCS lead which is less complex than known solutions. Particularly, the invention allows for shifting the resonant frequency of each conductor in the lead to ensure all conductors avoid resonance at MR frequencies.

Further features and embodiments of the present invention are described below with reference to the Figures, wherein

Fig. 1    shows a lead comprising co-radial conductors having electrical insulations, wherein each insulation can consist of one of at least two different materials; and

Fig. 2    shows a lead comprising an outer and a co-axial an inner coil structure, each structure comprising a plurality of co-radial conductors having electrical insulations, wherein each insulation can consist of one of at least two different materials.

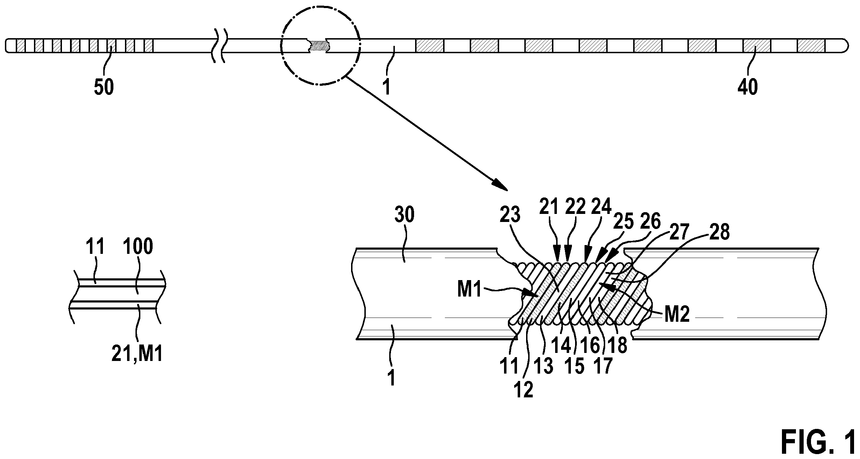

According to Fig. 1 a lead 1 according to the present invention comprises at least a first and a second conductor 12, 13, wherein the first conductor 12 and the second conductor 13 each comprise an electrically conducting core 100 that is surrounded by an electrical insulator 21 (as shown on the lower left side of Fig 1), wherein the electrical insulator 22 of the first conductor 12 consists of a first material M1, and wherein - analogously - the electrical insulator 23 of the second conductor 13 consists of a second material M2, wherein the first material M1 differs from the second material M2.

Furthermore, the lead 1 can comprises a lead body insulator 30 surrounding each individual conductor 12, 13 for providing further insulation and protection of the single conductors 12, 13.

Now, in order to avoid RF heating during MRI, conductors 11, 12,...,18 of lead 1 need inductance L and capacitance C. One way to incorporate inductance and capacitance into a lead 1 is to coil the conductors 11, 12 along the length of the lead 1 as shown in Fig. 1, which turns each conductor 11, 12 into a coil (or solenoid structure) with winding to winding parasitic capacitance. The LC combination creates a filter that blocks the high frequency MRI induced RF signals, while allowing the low frequency biological signals to pass unimpeded. Most SCS leads available today use straight conductors, which have very little inductance.

Particularly, the lead shown in Fig. 1 can be a co-radial SCS lead 1 with 8 electrodes 40 on a distal end of the lead 1, wherein each of the electrodes 40 is connected via one of the conductors 11, 18 to a connector contact 50 arranged on the proximal end of the lead 1. The close-up section in the bottom right of Fig. 1 shows the coiled conductors 11, 18. In this illustration three conductors 11, 12 and 14 are coated with an electrical insulator 21, 22, 24 consisting of a first material M1, while the five remaining conductors 13, 15, 16, 17, 18 are coated with an electrical insulator 23, 25, 26, 27, 28 consisting of a second material M2 that differs from the first material M1.

In addition to adding inductance and capacitance, resonance at MRI RF frequencies needs to be avoided in leads designed for MRI labeling. RF frequencies for MRI devices are e.g. ~64 MHz for 1.5 T machines, and ~128 MHz for 3T machines. If a conductor 11, 12 on the lead 1 shown in Fig. 1 resonates at MRI RF frequencies then the impedance of the lead 1 drops dramatically at these frequencies and very little energy is dissipated in the lead 1. The result is much more MRI induced current flowing through the lead 1 which results in much more RF heating at the electrodes 40. Avoiding resonance is particularly challenging for leads 1, particularly SCS leads 1, which typically have 8 different conductors 11, 18 connected to 8 different electrodes 40. Each conductor 11, 18 has its own resonance frequency which is distinct from the resonance frequency of the other conductors because each electrode 40 is typically located at a different distance along the lead 1, and hence the conductor 11, 18 connected to each electrode 40 has a different length. So, an 8 conductor lead 1 is much more challenging to design to avoid resonance than a typical 2 conductor cardiac lead because there are 4 times as many conductors that need to be designed to avoid resonance. Worse yet, some SCS leads incorporate paddle electrode arrays with 16 or more separate conductors connecting 16 or more different electrodes. These large number of electrodes 30 multiply the odds that at least one conductor 11, 18 connected to one electrode 40 will be in resonance at MRI frequencies.

To avoid such resonance frequencies, an embodiment of the present invention particularly uses coiled conductors 11, 18 for a lead 1, particularly an SCS lead 1, with at least two different materials M1, M2 as electrical insulator 21, 28 on the individual conductor core/wires 100 as shown e.g. in Fig. 1. The coiled conductors 11, 18 give inductance because the coiling creates a solenoid structure, and the coiling also gives capacitance due to increased conductor length, which is capacitive coupled to neighboring windings. The different insulators 21, 28 allow each conductor's self-resonance to be selected during the design phase to avoid MRI frequencies. Some conductors have material M1 as insulator material and some conductors have material M2 as insulator material. The inductance of a solenoid is approximated by the well-known equation $L = \mu_0 \frac{N^2 A}{l}$, the capacitance is approximated by $C = \frac{\varepsilon_0 \varepsilon_r A}{d}$, and the resonance frequency is approximated by $f = \frac{1}{2\pi\sqrt{LC}}$. The dielectric constant $\varepsilon_r$ is a function of the insulation material M1, M2 surrounding the respective conductor 11., 18. By changing the insulation material $\varepsilon_r$ changes, and hence the resonance frequency changes for the respective conductor M1, M2. The preferred materials M1, M2 for insulating the individual lead conductors 11., 18 are e.g. ETFE, PFA, and PTFE. The dielectric constant $\varepsilon_r$ for these materials are shown in Table 1 below. As can be seen ETFE, has a dielectric constant about 25% greater than PTFE or PFA. Therefore, in one embodiment ETFE is used on at least one conductor 11, 18, while PFA or PTFE are used on the other conductors.

Table 1:

|                     | ETFE | PFA | PTFE |
|---------------------|------|-----|------|
| Dielectric Constant | 2.6  | 2.1 | 2.1  |

In one embodiment 8 different conductors connected to 8 different electrodes are all wound co-radially as shown in Fig. 1.

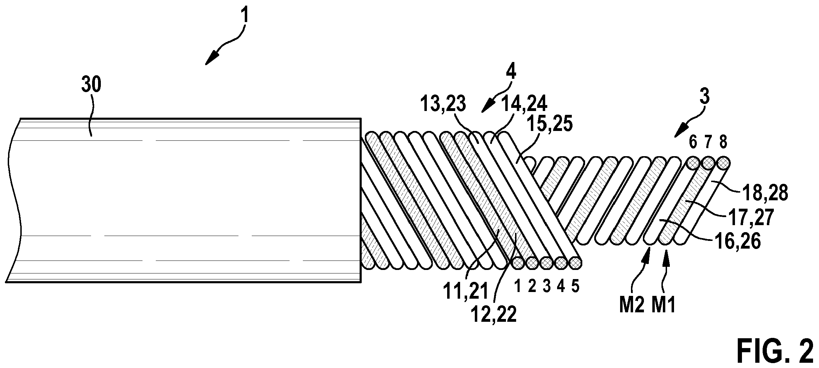

Fig. 2 shows a further embodiment of a lead 1 according to the present invention also using two different insulator materials M1, M2. Here, two conductors 11, 12 of an outer coil 4 structure comprised of 5 conductors 11, 12, 13, 14, 15 in form of co-radial helical coils comprise electrical insulators 21, 22 formed out of a first material M1. Further, the lead comprises an inner coil structure 3 surrounded by the co-axial outer coil structure 4, wherein the inner coil structure 3 is comprised of three conductors 16, 17, 18 in form of co-radial helical coils, wherein one conductor 17 is formed out of the first Material M1. The other conductors 13, 14, 15, and 16, 18 comprise insulators 23, 24, 25, 26, 28 made out of a different second material M2.

Here, in the second embodiment, a co-axial/co-radial design is used for the conductors 11, 18. In this embodiment there are two (or even more) layers of coiled conductors, namely an inner layer or coil structure 3 and an outer layer (or coil structure) 4. This construction is more complicated than the co-radial design shown in Figure 1, but it has several advantages. First, the outer coil 4 has less conductors than the co-radial design shown in Fig. 1 (since some of the conductors are moved to the inner coil 3), and less conductors means that each conductor has a finer pitch. The finer pitch results in a much greater inductance (inductance is proportional to the square of the pitch), which results in a better MRI induce RF current rejection. Second, the conductors 16, 18 in the inner coil structure 3 are shielded by the outer coil structure 4. Third, there is greater average capacitance between conductors in this design since the average distance between conductors 11, 18 is reduced. The reduced average distance is because of the finer pitch of both the inner and outer coil structure 3, 4 compared to the co-radial coil of Fig. 1, and the nested arrangement of the two coil structures 3, 4 which causes coupling between the coils structures 3, 4. The greater average capacitance results in a better (lower cut off frequency) low pass (LC) filter. In addition, the inductive and capacitive coupling mechanisms result in more current sharing between the conductors at MRI frequencies. This helps average out the MRI induced currents on each conductor, eliminating peaks. One example implementation of this co-axial/co-radial design shown in Fig. 2 is detailed in Table 2 below.

In another embodiment at least one conductor (e.g. of the conductors 11, 18 shown in Fig. 1 or 2) has no separate insulation around it at all (insulation to the body 30 is still provided by the lead body insulator/tube 30 which is typically silicone or polyurethane. This embodiment not only changes the resonance frequency of the conductor(s) with no separate insulation, but also allows all the conductors 11, 18 to be packaged closer together, resulting in a tighter winding pitch and higher overall inductance for each conductor. Particularly, at most, every other conductor can be without insulation.

Particularly, having different insulation materials M1, M2 on different filars of the inner and/or outer coil structure allows fine tuning of the resonant frequency of the lead 1 for each electrode 40. If a certain lead length leads to one or more electrodes 40 being in electrical resonance at MRI frequencies, then the insulation material M1, M2 can be changed on that particular filar/conductor to shift the resonance away from MRI frequencies. Furthermore, the present invention allows for visual identification between conductors during the manufacturing process (e.g. to make sure that the appropriate conductor gets welded to the appropriate contact). Particularly, colorants can be added to one or more of the coatings to make it readily apparent which conductor is which. Particularly, in the above-described embodiment in which the insulation is removed altogether on one or more filars/conductors, the pitch can be increased which increases the inductance and improves MRI performance. In one embodiment of this, only every other conductor is insulated.

**Claims**

1. Lead (1) comprising at least a first and a second conductor (12, 13), wherein the first conductor (13) and the second conductor (13) each comprise an electrically conducting core (100) that is surrounded by an electrical insulator (22, 23), wherein the electrical insulator (22) of the first conductor (12) consists of a first material (M1), and wherein the electrical insulator (23) of the second conductor (13) consists of a second material (M2), wherein the first material (M1) differs from the second material (M2).

2. Lead according to claim 1, wherein the first material (M1) and the second material (M2) are chosen such that the respective conductor (12, 13) comprises a resonance frequency that is different from a frequency used in an MRI device.

3. Lead according to claim 1 or 2, wherein the first material (M1) and the second material (M2) are selected from the group of the following materials: a ceramics, a polymer, ETFE, PFA, PTFE, polyimide, aluminum oxide, barium titanate, titanium dioxide.

4. Lead according to one of the preceding claims, wherein the first and the second conductor (12, 13) each form a helical coil.

5. Lead according to one of the preceding claims, wherein the lead (1) comprises a plurality of conductors (11, 12, ..., 18) including said first and said second conductor (12, 13), wherein each conductor (11, 12, ..., 18) of said plurality of conductors other than the first and the second conductor (12, 13) also comprises an electrically conducting core (100) that is surrounded by an electrical insulator (23, 24,...) formed out of the first material (M1) or out of the second material (M2), or out of a further material different from the first and the second material (M1, M2).

6. Lead according to one of the preceding claims, wherein the lead (1) comprises a lead body insulator (30) surrounding each individual conductor (11, 12,..., 18).

7. Lead according to claim 6, wherein the lead (1) comprises at least one conductor formed by a non-insulated electrically conducting member, which is electrically insulated with respect to its surrounding by means of electrical insulators (21, 22) of adjacent conductors (11, 12) of the lead (1) and/or by means of said lead body insulator (30).

8. Lead according to one of the preceding claims, wherein said first and said second conductor (12, 13) are co-radial and/or co-axial helical coils.

9. The lead according to claim 5 or one of the claims 6 to 8 when referring to claim 5, wherein said plurality of conductors (11, 12) form an inner coil structure (3) and a co-axial outer coil structure (4) surrounding the inner coil structure (3), wherein each coil structure (3, 4) comprises at least one conductor or several conductors of said plurality of conductors (11, 12, 18).

10. Lead according to one of the preceding claims, wherein the lead (1) is a medical lead.

11. Lead according to one of the preceding claims, wherein the lead (1) is an electrode lead (1) comprising a plurality of electrodes (40).

12. Lead according to one of the preceding claims, wherein the lead (1) is adapted for spinal cord stimulation.

13. Method for producing a lead, wherein a at least a first and a second conductor (12, 13) are provided, wherein the first conductor (12) and the second conductor (13) each comprise an electrically conducting core (100) that is surrounded by an electrical insulator (21), wherein the electrical insulator (22) of the first conductor (12) is made out of a first material (M1), and wherein the electrical insulator (23) of the second conductor (13) is made out of a second material (M2), wherein the first material (M1) differs from the second material (M2), and wherein said conductors (12, 13) are surrounded by an outer lead body insulator (30).

**14.** Method according to claim 13, wherein the first material (M1) and the second material (M2) are chosen such that the respective conductor (11, 12) comprises a resonance frequency that is different from a given frequency used in an MRI device.

FIG. 1

EP 3 560 550 A1

FIG. 2

EP 3 560 550 A1

**EP 3 560 550 A1**

Europäisches Patentamt
European Patent Office
Office européen des brevets

# EUROPEAN SEARCH REPORT

Application Number

EP 18 17 2976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/245734 A1 (MCDONALD MATTHEW LEE [US] ET AL) 19 September 2013 (2013-09-19) * paragraphs [0039] - [0049]; claims 1, 11; figures 1-4 * | 1-14 | INV.<br>A61N1/08<br>A61N1/05<br><br>ADD.<br>G01R33/28<br>H01B7/04<br>H03H1/00 |
| X,D | US 9 399 129 B2 (PACESETTER INC) 26 July 2016 (2016-07-26) * column 2, lines 48-67; figures 1-2 * * column 8, line 17 - column 10, line 50; figures 5, 7 * * column 11, line 63 - column 12, line 25 * | 1,3-6, 8-11,13 | |
| A | US 2014/052203 A1 (BULKES CHERIK [US]) 20 February 2014 (2014-02-20) * paragraphs [0039] - [0050]; figures 1-6 * | 1-14 | |
| A | US 2015/314123 A1 (SHARMA VIVEK [US] ET AL) 5 November 2015 (2015-11-05) * paragraphs [0046] - [0050], [0059] - [0071]; figures 1-6D * | 1-14 | |

TECHNICAL FIELDS SEARCHED (IPC)

A61N
G01R
H01B
H03H

The present search report has been drawn up for all claims

| Place of search | Date of completion of the search | Examiner |
|---|---|---|
| Munich | 30 October 2018 | Fischer, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 560 550 A1**

## ANNEX TO THE EUROPEAN SEARCH REPORT
## ON EUROPEAN PATENT APPLICATION NO.

EP 18 17 2976

This annex lists the patent family members relating to the patent documents cited in  the above-mentioned European search report.
The members are as contained in the European Patent Office EDP file on
The European Patent Office is in no way liable for these particulars which are merely given for the purpose of information.

30-10-2018

| Patent document cited in search report | | | Publication date | Patent family member(s) | | | Publication date |
|---|---|---|---|---|---|---|---|
| US 2013245734 | A1 | | 19-09-2013 | NONE | | | |
| US 9399129 | B2 | | 26-07-2016 | NONE | | | |
| US 2014052203 | A1 | | 20-02-2014 | NONE | | | |
| US 2015314123 | A1 | | 05-11-2015 | AU | 2015252868 | A1 | 08-12-2016 |
| | | | | EP | 3137162 | A1 | 08-03-2017 |
| | | | | US | 2015314123 | A1 | 05-11-2015 |
| | | | | WO | 2015168645 | A1 | 05-11-2015 |

EPO FORM P0459

For more details about this annex : see Official Journal of the European Patent Office, No. 12/82

**EP 3 560 550 A1**

**Patent documents cited in the description**

- US 9399129 B
- US 20160331960 A1